# EUROPÄISCHE PATENTSCHRIFT

(11) **EP 3 461 519 B1**
(45) Veröffentlichungstag und Bekanntmachung des Hinweises auf die Patenterteilung: **25.10.2023**
(21) Anmeldenummer: 18191673.5
(22) Anmeldetag: 30.08.2018
(51) Int. Cl.: A61M 5/31, A61M 5/315, A61M 5/50

(54) **MEDIZINISCHE INJEKTIONSVORRICHTUNG**
MEDICAL INJECTION DEVICE
DISPOSITIF D'INJECTION MÉDICAL

(30) Priorität: 27.09.2017 DE 102017217236
(43) Veröffentlichungstag der Anmeldung: 03.04.2019
(73) Patentinhaber: B. Braun Melsungen AG, 34212 Melsungen (DE)
(72) Erfinder: Hartung, Jürgen, 34298 Helsa (DE); Kiehle, Christin, 34121 Kassel (DE); Klute, Volker, 34212 Melsungen (DE); Koot, Jan, 34576 Homberg (DE)
(74) Vertreter: Patentanwälte Ruff, Wilhelm, Beier, Dauster & Partner mbB

(56) Entgegenhaltungen:
- EP-A1- 2 153 856
- WO-A1-92/04064
- WO-A1-93/23099

## Beschreibung

Die Erfindung betrifft eine medizinische Injektionsvorrichtung gemäß dem Oberbegriff des Anspruchs 1.

Eine derartige Injektionsvorrichtung ist aus der WO 92/04064 A1 bekannt.

Eine weitere Injektionsvorrichtung ist aus der EP 2 153 856 B1 bekannt. Die bekannte Injektionsvorrichtung weist einen Hohlzylinder mit einem Hohlraum zur Aufnahme einer zu verabreichenden Flüssigkeit auf. Der Hohlraum ist fluidleitend mit einem distal angeordneten Durchlass verbunden. Weiter weist die Injektionsvorrichtung einen an dem Hohlzylinder ausgebildeten Verbindungsabschnitt in Form eines männlichen Luer-Abschnittes auf. Dieser ist lösbar fluiddicht mit einem weiblichen Luer-Abschnitt einer als Zusammenbau bezeichneten Injektionskomponente verbunden. Die Injektionskomponente weist an ihrem distalen Ende eine Injektionsnadel auf. Der Durchlass ist fluidleitend mit einem als Ausnehmung bezeichneten Lumen der Injektionskomponente verbunden. Mittels Einwirkung eines in dem Hohlzylinder in distale Richtung bewegten Kolbens kann Flüssigkeit ausgehend von dem Hohlraum über den Durchlass durch das Lumen aus der Injektionsnadel gepresst und somit einem Patienten verabreicht werden. Um eine nochmalige Verwendung der Injektionsvorrichtung zu unterbinden und somit der Übertragung von Krankheitserregern vorzubeugen, ist eine Sperrventilanordnung vorgesehen. Diese weist einen weichelastischen Dichtring mit einer Öffnung und einen Ventilkörper auf. Beim Verabreichen der Flüssigkeit wird der Ventilkörper mittels der hierbei distal gerichteten Strömung der Flüssigkeit ausgehend von einer Freigabeposition, in welcher der Durchlass freigegeben ist, in distaler Richtung durch die Öffnung des Dichtringes in eine Sperrposition bewegt. In dieser hintergreift der Ventilkörper die Öffnung derart, dass eine Bewegung des Ventilkörpers in die Freigabeposition gesperrt und der Durchlass gegen eine proximal gerichtete Strömung abgedichtet ist. Auf diese Weise ist die bekannte Injektionsvorrichtung gegen ein nochmaliges Aufziehen des Hohlraumes mit Flüssigkeit gesichert. Bei der bekannten Injektionsvorrichtung ist die Sperrventilanordnung in der Ausnehmung der Injektionskomponente angeordnet, was eine dementsprechende Ausgestaltung der Injektionskomponente erforderlich macht.

Aufgabe der Erfindung ist es, eine Injektionsvorrichtung der eingangs genannten Art zu schaffen, die einen vereinfachten Aufbau aufweist und insbesondere eine Verwendung in Verbindung mit einer standardisierten Injektionskomponente, insbesondere einer Einwegkanüle, ermöglicht.

Diese Aufgabe wird durch eine medizinische Injektionsvorrichtung mit den Merkmalen des Anspruchs 1 gelöst. Durch die erfindungsgemäße Lösung kann insbesondere auf eine Anordnung der Sperrventilanordnung an der Injektionskomponente und insoweit auf eine spezielle diesbezügliche Ausgestaltung der Injektionskomponente verzichtet werden. Denn erfindungsgemäß ist ein Adapterkörper vorgesehen, der einends, nämlich an seinem distalen Ende, zur lösbaren Verbindung mit einer, vorzugsweise standardisierten, Injektionskomponente vorgesehen und andernends, nämlich an seinem proximalen Ende, mit dem Hohlzylinder verbunden ist. Der Adapterkörper bildet insoweit eine Art Übergangsstück zwischen dem Hohlzylinder und der Injektionskomponente. Hierbei ist die Sperrventilanordnung erfindungsgemäß am proximalen Ende des Adapterkörpers und dem distalen Ende des Hohlzylinders angeordnet, so dass die Injektionsvorrichtung in Verbindung mit einer üblichen - im Sinne von nicht mit einer Sperrventilanordnung ausgestatteten - Injektionskomponente verwendbar ist. Zudem sind der Hohlzylinder und der Adapterkörper erfindungsgemäß als separat voneinander gefertigte Bauelemente ausgebildet, so dass insbesondere der Verbindungsabschnitt des Adapterkörpers unabhängig von dem Hohlzylinder ausgestaltet sein kann und umgekehrt. Auf diese Weise können fertigungsseitige Vorteile erreicht werden. Der Adapterkörper ist an seinem proximalen Ende vorzugsweise mittels einer formschlüssigen Rast- und/oder einer reibschlüssigen Klemmverbindung mit dem Hohlzylinder verbunden. Die derartige Verbindung kann lös- oder bevorzugt unlösbar gestaltet sein. Der Adapterkörper ist beispielsweise als Kunststoffspritzgussbauteil gefertigt. Der Verbindungsabschnitt kann beispielsweise in Form eines als solchen grundsätzlich bekannten, vorzugsweise männlichen, Luer-Slip- oder Luer-Lock-Abschnittes ausgebildet sein. Alternativ ist es möglich, dass der Verbindungsabschnitt eine andersartige, vorzugsweise standardisierte, Ausgestaltung aufweist. Der Verbindungsabschnitt ist zur Verbindung des Adapterkörpers und damit der Injektionsvorrichtung mit einem komplementär ausgestalteten Gegenverbindungsabschnitts einer beispielsweise in Form einer Kanüle, eines Schlauches oder dergleichen gestalteten Injektionskomponente vorgesehen. Die Sperrventilanordnung ist vorteilhafterweise eigenmediumbetätigt, so dass eine Sperrung der Injektionsvorrichtung gegen eine nochmalige Verwendung mittels einer im Wesentlichen vollständigen Entleerung der Flüssigkeit durch den Durchlass bewirkbar ist und keiner weiteren Handlung bedarf. Die Sperrventilanordnung ist vorzugsweise abschnittsweise an dem Hohlzylinder und abschnittsweise an dem Adapterkörper festgelegt. Beispielsweise kann der Dichtring am proximalen Ende des Adapterkörpers und der Ventilkörper am distalen Ende des Hohlzylinders festgelegt sein oder umgekehrt. Die Begriffe proximal und distal bezeichnen eine in Bezug auf einen Referenzpunkt nähere bzw. weiter entfernte Lage.

Weiter gemäß der Erfindung weist der Hohlzylinder einen einstückig angeformten Zylinderboden auf, wobei der Ventilkörper an einer durch den Zylinderboden erstreckten Führungsbahn axial beweglich geführt ist. Die Führungsbahn kann in Form einer, vorzugsweise kreiszylindrischen, Durchgangsbohrung, einer Ausnehmung oder dergleichen durch den Zylinderboden erstreckt sein. Der Ventilkörper weist zum Zwecke der Führung an der Führungsbahn vorzugsweise einen Schaftabschnitt auf. Sofern die Führungsbahn als kreiszylindrische Bohrung gestaltet ist, kann der Schaftabschnitt bolzenförmig gestaltet und gleitbeweglich in die Durchgangsbohrung eingepasst sein. Der Zylinderboden begrenzt den Hohlraum in distaler Richtung. Vorzugsweise ist der Durchlass durch den Zylinderboden erstreckt.

In weiterer Ausgestaltung der Erfindung weist der Dichtring einen Klemmabschnitt auf, der zwischen dem Hohlzylinder, insbesondere dem Zylinderboden, und dem Adapterkörper in Axialrichtung und/oder in Radialrichtung formschlüssig festgelegt, insbesondere geklemmt, ist. Der Klemmabschnitt ist vorzugsweise an einem proximalen Stirnende des Dichtringes angeordnet und kann beispielsweise in Form eines Radialbundes, eines Kragens oder dergleichen ausgebildet sein. Vorzugsweise ist der Klemmabschnitt zwischen dem distalen Ende des Hohlzylinders und dem proximalen Ende des Adapterkörpers formschlüssig festgelegt. Diese Ausgestaltung der Erfindung erlaubt eine besonders einfache Montage der Injektionsvorrichtung.

In weiterer Ausgestaltung der Erfindung weist das Lumen des Adapterkörpers einen proximal angeordneten Passabschnitt auf, in den der Dichtring eingepasst ist. Das Lumen ist in Form einer in Axialrichtung durch den Adapterkörper erstreckten Durchgangsöffnung ausgebildet. Der Passabschnitt des Lumens kann gegenüber einem weiteren Verlauf des Lumens in radialer Richtung aufgeweitet oder verengt sein. Eine besonders einfache Einpassbarkeit des Dichtringes wird erreicht, wenn der Passabschnitt an einem proximalen Stirnendbereich des Lumens angeordnet ist.

In weiterer Ausgestaltung der Erfindung weist der Passabschnitt eine einseitig in proximale Richtung offene, hinterschnittene Radialnut auf, wobei der Dichtring die Radialnut hintergreift und somit in proximaler Richtung formschlüssig an dem Adapterkörper festgelegt ist. Aufgrund der derartigen formschlüssigen Verbindung des Dichtringes mit dem Passabschnitt kann dieser bei der Fertigung der Injektionsvorrichtung vor einem Verbinden des Adapterkörpers mit dem Hohlzylinder verliersicher an dem Adapterkörper festgelegt werden. Dies erlaubt eine einfache Handhabung während einer Endmontage der Injektionsvorrichtung, genauer: bei einem Verbinden des Hohlzylinders mit dem Adapterkörper.

In weiterer Ausgestaltung der Erfindung weist das distale Ende des Hohlzylinders einen Rastabschnitt auf, der derart mit einem komplementären Gegenrastabschnitt des Adapterkörpers, vorzugsweise unlösbar, verrastet ist, dass der Adapterkörper in Axialrichtung an dem Hohlzylinder festgelegt ist. Der Rastabschnitt kann eine oder mehrere Rastnasen, -haken oder -zähne und der Gegenrastabschnitt dementsprechend komplementär gestaltete Rastöffnungen, -nuten oder dergleichen aufweisen oder umgekehrt. Mittels der derartigen Rastverbindung sind der Hohlzylinder und der Adapterkörper wenigstens in Axialrichtung fest miteinander verbunden, so dass einem unbeabsichtigten Abziehen des Adapterkörpers von dem Hohlzylinder oder umgekehrt entgegengewirkt ist.

In weiterer Ausgestaltung der Erfindung weist das distale Ende des Hohlzylinders einen Profilabschnitt, vorzugsweise in Form einer Vielverzahnung auf, der derart mit einem komplementären Gegenprofilabschnitt des Adapterkörpers verzahnt ist, dass der Adapterkörper in Umfangsrichtung an dem Hohlzylinder festgelegt ist. Demzufolge ist eine Verdrehsicherung zwischen dem Hohlzylinder und dem Adapterkörper erreicht. Auf diese Weise kann eine verbesserte Handhabung der Injektionsvorrichtung ermöglicht werden.

In weiterer Ausgestaltung der Erfindung ist der Rastabschnitt und/oder der Profilabschnitt an einem umlaufenden und distal zu dem Hohlraum, insbesondere dem Zylinderboden, erstreckten Kragenabschnitt des Hohlzylinders ausgebildet. Der Kragenabschnitt ist vorzugsweise einstückig mit dem Hohlzylinder ausgebildet. Vorzugsweise weist der Kragenabschnitt einen runden, bevorzugt kreisförmigen, Querschnitt auf. Der Kragenabschnitt kann als ein durchgehender, zusammenhängender Wandungsabschnitt gefertigt sein oder alternativ durch voneinander getrennte, einzelne Wandabschnitte gebildet sein. Eine einfache Handhabung und Fertigbarkeit der Injektionsvorrichtung wird erreicht, wenn der Kragenabschnitt parallel, vorzugsweise koaxial, zu einer Mantelwandung des Hohlzylinders orientiert ist.

Weiter gemäß der Erfindung weist der Ventilkörper an seinem distalen Ende einen radial aufgeweiteten Kopfabschnitt auf. Der Kopfabschnitt kann insbesondere eine kugel- oder pilzkopfförmige Grundform aufweisen. Die Abmessungen des Kopfabschnittes sind vorteilhafterweise derart auf den Durchmesser der Öffnung des Dichtringes abgestimmt, dass der Kopfabschnitt mittels einer distal durch den Durchlass gerichteten Strömung der Flüssigkeit ausgehend von der Freigabeposition durch die Öffnung des Dichtringes beweglich ist und diese in der Sperrposition formschlüssig hintergreift.

In weiterer Ausgestaltung der Erfindung weist der Kopfabschnitt einen in distale Richtung erstreckten und radial verjüngten Fortsatz auf. Der Fortsatz kann stirnendseitig mit einer Anlaufschrägung versehen sein. Mittels des Fortsatzes kann insbesondere eine verbesserte Zentrierung des Ventilkörpers in der Öffnung des Dichtringes erreicht werden.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer, teilweiser geschnittener Seitenansicht eine Ausführungsform einer erfindungsgemäßen Injektionsvorrichtung, die lösbar mit einer Injektionskomponente in Form einer Einwegkanüle verbunden ist,
- Fig. 2: in schematischer Längsschnittdarstellung die Injektionsvorrichtung nach Fig. 1 im Bereich einer Sperrventilanordnung,
- Fig. 3: eine weitere Ausführungsform einer erfindungsgemäßen Injektionsvorrichtung in einer Längsschnittdarstellung entsprechend Fig. 2,
- Fig. 4: in einer schematischen, perspektivischen Explosionsdarstellung die Infusionsvorrichtung nach den Fig. 1 und 2 im Bereich der Sperrventilanordnung,
- Fig. 5: in einer schematischen Querschnittsdarstellung entlang einer Schnittlinie A-A gemäß Fig. 2 die Injektionsvorrichtung nach den Fig. 1, 2 und 4,
- Fig. 6 bis 8: jeweils in abgeschnittener Längsschnittdarstellung die Sperrventilanordnung der Injektionsvorrichtungen nach den Fig. 1 bis 5 in unterschiedlichen Positionen bei einer Verlagerung von einer Freigabe- in eine Sperrposition,
- Fig. 9: in einer abgeschnittenen schematischen Längsschnittdarstellung eine weitere Ausführungsform einer erfindungsgemäßen Injektionsvorrichtung und
- Fig. 10: in einer Darstellung entsprechend Fig. 9 eine weitere Ausführungsform einer erfindungsgemäßen Injektionsvorrichtung.

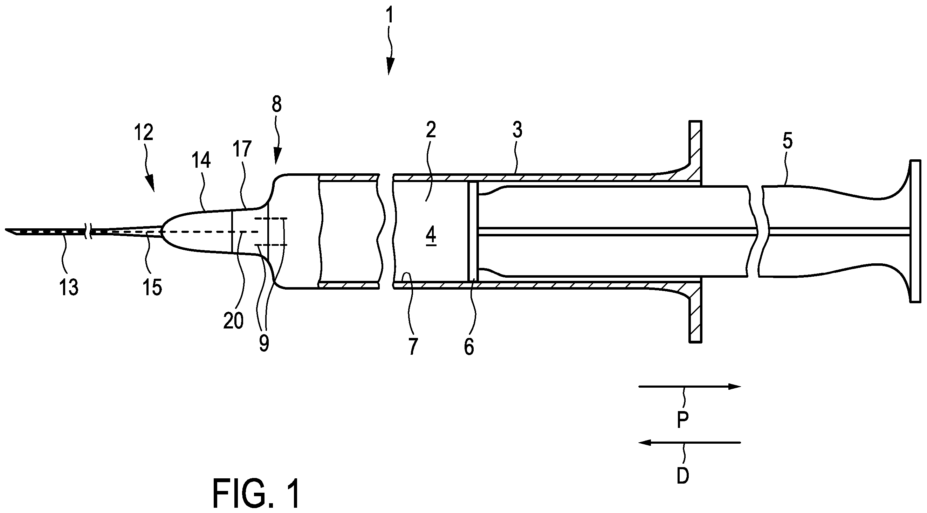

Eine medizinische Injektionsvorrichtung 1 nach Fig. 1 ist in Form einer Einwegspritze gestaltet und zur Einmalverwendung bei der Verabreichung einer medizinischen Flüssigkeit 2 vorgesehen.

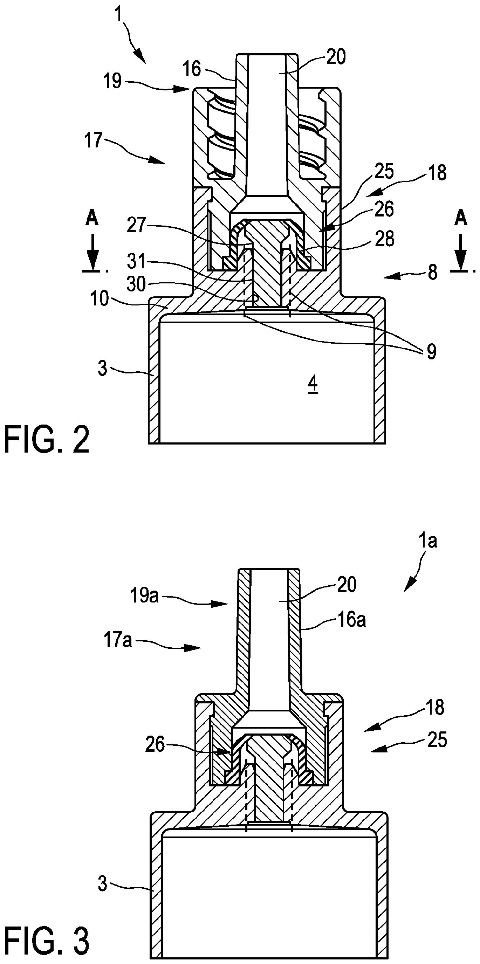

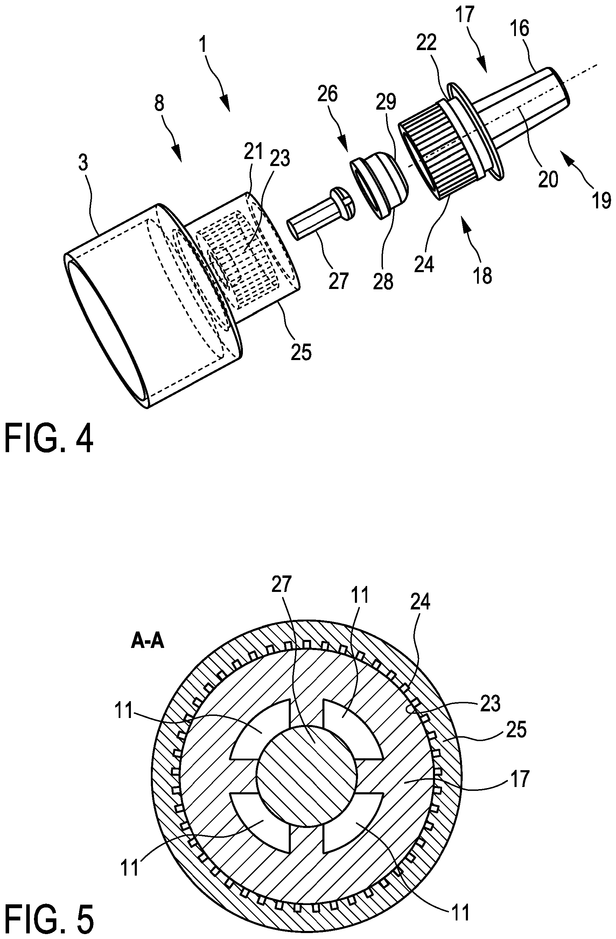

Die Injektionsvorrichtung 1 weist einen Hohlzylinder 3 mit einem Hohlraum 4 zur Aufnahme der Flüssigkeit 2 auf. Zur manuellen Betätigung der Injektionsvorrichtung ist ein Betätigungsstempel 5 mit einem stirnendseitig angeordneten Kolben 6 vorgesehen. Der Kolben 6 dichtet den Hohlraum 4 in proximaler Richtung P fluiddicht ab und ist gegenüber einer Innenwandung 7 des Hohlzylinders 3 in Axialrichtung gleitbeweglich. Am distalen Ende 8 des Hohlzylinders 3 ist ein Durchlass 9 angeordnet, der anhand Fig. 1 lediglich mittels zweier strichlierter Linien angedeutet ist. Der Durchlass 9 ist fluidleitend mit dem Hohlraum 4 verbunden und zum Ein- und Ausströmen der Flüssigkeit 2 aus dem Hohlraum 4 vorgesehen. Wie insbesondere anhand Fig. 2 ersichtlich ist, erstreckt sich der Durchlass 9 durch einen einstückig an den Hohlzylinder 3 angeformten Zylinderboden 10, der den Hohlraum 4 in distaler Richtung D begrenzt. Anhand der Querschnittsdarstellung der Fig. 5 ist ersichtlich, dass der Durchlass 9 aus vier Öffnungen 11 gebildet ist. Die Öffnungen 11 sind in Umfangsrichtung jeweils um 90° versetzt zueinander und konzentrisch zu einer Mittellängsachse des Hohlzylinders 3 angeordnet und weisen jeweils eine kreisringsegmentförmige Gestalt auf. Die Injektionsvorrichtung 1 ist an ihrem distalen Ende mit einer Injektionskomponente in Form einer Einwegkanüle 12 verbunden. Die Einwegkanüle 12 weist eine hypodermische Injektionsnadel 13 und einen Nadelansatz 14 auf. Die Einwegkanüle 12 ist auf grundsätzlich bekannte Weise gestaltet und weist insbesondere ein durchgängiges, anhand Fig. 1 strichliert angedeutetes, Lumen 15 auf. Das Lumen 15 der Einwegkanüle 12 ist auf noch näher zu beschreibende Art und Weise mit dem Durchlass 9 und dem Hohlraum 4 fluidleitend verbunden. Zur Verbindung mit der Einwegkanüle 12 weist die Injektionsvorrichtung 1 einen Verbindungsabschnitt 16 (Fig. 2) auf. Der Verbindungsabschnitt 16 ist in Form eines männlichen Luer-Lock-Abschnittes gestaltet und auf grundsätzlich bekannte, jedoch nicht näher dargestellte Art und Weise mit einem in Form eines weiblichen Luer-Lock-Abschnittes gestalteten Gegenverbindungsabschnitt der Einwegkanüle 12, der an dem Nadelansatz 14 angeordnet ist, lösbar fluiddicht verbunden.

Wie insbesondere anhand der Fig. 2 und 4 ersichtlich ist, ist ein Adapterkörper 17 mit einem proximalen Ende 18, das kraft- und/oder formschlüssig mit dem distalen Ende 8 des Hohlzylinders 3 verbunden ist, und einem distalen Ende 19, an dem der Verbindungsabschnitt 16 ausgebildet ist, vorgesehen. Weiter weist der Adapterkörper 17 ein Lumen 20 in Form einer durchgängig erstreckten Öffnung auf. Das Lumen 20 ist einends fluidleitend mit dem Lumen 15 der Einwegkanüle 12 und andernends auf noch näher zu beschreibende Weise fluidleitend mit dem Durchlass 9 und somit dem Hohlraum 4 verbunden. Der Adapterkörper 17 ist als ein separat von dem Hohlzylinder 3 gefertigtes Bauteil ausgebildet, vorzugsweise als Kunststoffspritzgussbauteil, und mittels einer formschlüssigen Rast- sowie einer Vielzahnverbindung mit dem Hohlzylinder 3 verbunden. Hierfür weist das distale Ende 8 des Hohlzylinders 3 einen Rastabschnitt 21 auf. Der Rastabschnitt 21 ist mit einem komplementären Gegenrastabschnitt 22 des Adapterkörpers 17 verrastet, so dass der Adapterkörper 17 in Axialrichtung an dem Hohlzylinder 3 festgelegt ist (Fig. 4). Der Rastabschnitt 21 ist in Form einer in Umfangsrichtung umlaufenden Rastnase ausgestaltet. Hierzu komplementär weist der Adapterkörper 17 eine in Umfangsrichtung umlaufende Rastnut als Gegenrastabschnitt 22 auf. Die Vielzahnverbindung ist zwischen einem am distalen Ende 8 des Hohlzylinders 3 ausgebildeten Profilabschnitt 23 und einem komplementären Gegenprofilabschnitt 24 des Adapterkörpers 17 ausgebildet. Auf diese Weise ist der Adapterkörper 17 in Umfangsrichtung an dem Hohlzylinder 3 festgelegt und gegen ein ungewolltes Verdrehen gesichert. Der Profilabschnitt 23 ist in Form einer in Umfangsrichtung durchgängigen Vielverzahnung mit einer Vielzahl von in Axialrichtung erstreckten Längsnuten ausgebildet. Dementsprechend ist der Gegenprofilabschnitt 24 in Form einer abmesserseitig auf die Innenverzahnung des Profilabschnittes 23 abgestimmte Außenverzahnung ausgebildet.

Sowohl der Rastabschnitt 21 als auch der Profilabschnitt 23 sind an einem umlaufenden und distal zu dem Hohlraum 4, genauer: dem Zylinderboden 10, erstreckten Kragenabschnitt 25 des Hohlzylinders 3 ausgebildet. Der Kragenabschnitt 25 erstreckt sich in Form einer Zylinderbuchse, die an ihrem distalen Ende in Axialrichtung offen und an ihrem proximalen Ende durch den Zylinderboden 10 begrenzt ist. Der Kragenabschnitt 25 weist einen kreiszylindrischen Querschnitt auf und ist im Wesentlichen koaxial zu einer Mittellängsachse des Hohlzylinders 3 orientiert. Weiter weist der Kragenabschnitt 25 im Vergleich zu dem Hohlzylinder 3 einen geringeren Durchmesser auf und ist einstückig mit dem Zylinderboden 10 und insoweit einstückig mit dem Hohlzylinder 3 ausgebildet.

Weiter weist die Injektionsvorrichtung 1 eine Sperrventilanordnung 26 mit einem Ventilkörper 27 und einem weichelastischen Dichtring 28 mit einer Öffnung 29 auf. Die Sperrventilanordnung 26 ist an dem proximalen Ende 18 des Adapterkörpers 17 und an dem distalen Ende 8 des Hohlzylinders 3 angeordnet.

Der Ventilkörper 27 ist an einer durch den Zylinderboden 10 erstreckten Führungsbahn 30 axialbeweglich geführt. Die Führungsbahn 30 ist in Form einer kreiszylindrischen Bohrung in Axialrichtung durch den Zylinderboden 10 erstreckt. Der Innendurchmesser der Führungsbahn 30 ist derart auf einen Außendurchmesser eines Schaftabschnittes 31 des Ventilkörpers 27 abgestimmt, dass dieser leichtgängig und im Wesentlichen fluiddicht in die Führungsbahn 30 eingepasst ist.

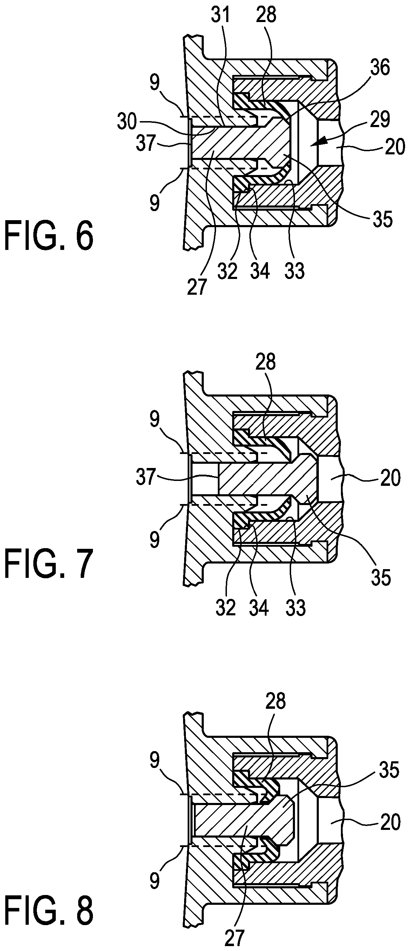

Der Dichtring 28 weist - jedenfalls in seiner insbesondere anhand Fig. 2 ersichtlichen undeformierten Konfiguration - eine kappen- oder glockenförmige Grundform auf. Sowohl in Axial- als auch in Radialrichtung ist der Dichtring 28 formschlüssig zwischen dem Hohlzylinder 3, genauer: dem Zylinderboden 10, und dem Adapterkörper 17 formschlüssig festgelegt. Zu diesem Zweck weist der Dichtring 28 einen Klemmabschnitt 32 auf. Der Klemmabschnitt 32 ist insbesondere anhand der Fig. 6 bis 8 näher ersichtlich und an einem proximalen Ende des Dichtringes 28 in Form eines Radialbundes ausgebildet. Zur Anordnung des Dichtringes 28 an dem Adapterkörper 17 ist dessen proximales Ende 18 dementsprechend gestaltet. So weist das Lumen 20 des Adapterkörpers 17 einen Passabschnitt 33 auf. Der Passabschnitt 33 ist gegenüber einem weiteren Verlauf des Lumens 20 in radialer Richtung zur Aufnahme des Dichtringes 28 und weist zudem eine einseitig in proximale Richtung offene Radialnut 34 auf. Der Klemmabschnitt 32 des Dichtringes 28 aufgeweitet ist in die Radialnut 34 des Passabschnittes 33 eingepasst.

Wie weiter insbesondere anhand der Fig. 6 bis 8 ersichtlich ist, weist der Ventilkörper 27 an seinem distalen Ende einen radial gegenüber dem Schaftabschnitt 31 aufgeweiteten Kopfabschnitt 35 auf. Der Kopfabschnitt 35 ist pilzkopfförmig ausgestaltet und hinsichtlich seines Außendurchmessers auf den Innendurchmesser der Öffnung 29 des Dichtringes 28 abgestimmt. Insbesondere anhand der Fig. 6 bis 8 wird nachfolgend näher auf die Funktion der Sperrventilanordnung 26 und das Zusammenwirken des Ventilkörpers 27 mit dem Dichtring 28 eingegangen.

Ausgehend von der anhand Fig. 6 ersichtlichen Konfiguration der Sperrventilanordnung 26, die als Freigabeposition bezeichnet wird, kann die Injektionsvorrichtung 1 auf grundsätzlich bekannte Weise mittels einer in proximaler Richtung P orientierten Bewegung des Betätigungsstempels 5 mit Flüssigkeit befüllt bzw. aufgezogen werden. Hierbei wird der Kolben 6 gegenüber der Innenwandung 7 des Hohlzylinders 3 in proximale Richtung P bewegt und Flüssigkeit ausgehend von einem nicht näher ersichtlichen Flüssigkeitsreservoir, das mit dem Lumen 20 des Adapterkörpers 17 fluidleitend verbunden ist, durch das Lumen 20, die Öffnung 29, einen zwischen dem Kopfabschnitt 35 des Ventilkörpers 27 und dem Dichtring 28 ausgebildeten Radialspalt 36, in die Öffnungen 11 des Durchlasses 9 und weiter in den Hohlraum 4 des Hohlzylinders 3 gesaugt. Hierbei verbleibt der Ventilkörper 27 auf einer dem Durchlass 9 zugewandten Seite der Öffnung 29, die nicht wesentlich von der anhand Fig. 6 ersichtlichen Position abweicht.

Zum Entleeren der Injektionsvorrichtung 1 wird der Kolben 6 über den Betätigungsstempel 5 in distale Richtung D bewegt und somit der Hohlraum 4 druckbeaufschlagt. Aufgrund dieser Druckbeaufschlagung gelangt die Flüssigkeit 2 aus dem Hohlraum 4 über die Öffnungen 11 des Durchlasses 9 und den Radialspalt 36 in das Lumen 20 des Adapterkörpers 17. Soweit die Einwegkanüle 12 mit dem Adapterkörper 17 verbunden ist - wie exemplarisch anhand Fig. 1 dargestellt - gelangt die Flüssigkeit 2 weiter von dem Lumen 20 in das Lumen 15 bis zu einer Austrittsöffnung der Injektionsnadel 13 und kann derart einen Patienten verabreicht werden. Hierbei ist die Sperrventilanordnung 26 derart gestaltet, dass der Ventilkörper 27 mittels einer solchen distal gerichteten Strömung der Flüssigkeit 2 ausgehend von der anhand Fig. 6 ersichtlichen Freigabeposition durch die Öffnung 29 des Dichtringes 28 bewegt wird. Diese Axialbewegung des Ventilkörpers 27 in distale Richtung D wird durch eine Druckbeaufschlagung eines proximalen Stirnendes 37 und axial projizierten, dem Durchlass 9 zugewandten Fläche des Kopfabschnittes 35 bewirkt. Infolge der weichelastischen Ausgestaltung des Dichtringes 28 weitet sich die Öffnung 29 bei einer derartigen Bewegung des Ventilkörpers 27 zunächst auf und liegt nach einem vollständigen Durchtritt des Kopfabschnittes 35 an dem Schaftabschnitt 31 des Ventilkörpers 27 an. In einer solchen anhand Fig. 7 verdeutlichten Sperrposition hintergreift der Ventilkörper 27 demnach die Öffnung 29. Demzufolge wird ein erneutes Aufziehen der Injektionsvorrichtung 1 mit Flüssigkeit verhindert. Denn ausgehend von der anhand Fig. 7 ersichtlichen Konfiguration wird der Ventilkörper 27 infolge einer proximal gerichteten Verlagerung des Kolbens 6 unter druckbedingt - in Bezug auf die Zeichenebene der Fig. 6 bis 8 - nach links angesaugt. Hierbei wird zum einen der Durchlass 9 infolge eines Dichtkontaktes zwischen dem Dichtring 28 und dem Kopfabschnitt 35 abgedichtet. Zum anderen wird eine weitere Bewegung des Ventilkörpers 27 in proximale Richtung P mittels des Dichtringes 28 unterbunden. Hierbei wird der Dichtring 28 weichelastisch deformiert und nach innen gewölbt und bildet eine formschlüssige Verbindung mit dem Kopfabschnitt 35 aus. Auf diese Weise wird eine nochmalige Verwendung der Injektionsvorrichtung 1 verhindert.

Weitere Ausführungsformen erfindungsgemäßer Injektionsvorrichtungen 1a, 1b, 1c sind anhand der Fig. 3, 9 bzw. 10 ersichtlich. Die Ausführungsformen nach den Fig. 3, 9 sowie 10 entsprechen im Wesentlichen der zuvor anhand der Fig. 1, 2 sowie 4 bis 8 beschriebenen Ausführungsform. Zur Vermeidung von Wiederholungen wird daher auf die Offenbarung zu der Ausführungsform nach den letztgenannten Figuren verwiesen. Nachfolgend wird lediglich auf die wesentlichen Unterschiede der jeweiligen Ausführungsform nach den Fig. 3, 9 bzw. 10 eingegangen. Bauteile und Abschnitte, die bei den Injektionsvorrichtungen 1a bis 1c identisch zu der Injektionsvorrichtung 1 sind, sind insoweit mit identischen Bezugszeichen versehen. Funktionsgleiche Bauteile und Abschnitte, die jedoch in ihrer konstruktiven Ausführung unterschiedlich sind, sind mit gleichen Bezugszeichenziffern unter Hinzufügung von Kleinbuchstaben bezeichnet.

Die Injektionsvorrichtung 1a nach Fig. 3 unterscheidet sich von der Injektionsvorrichtung 1 (Fig. 2) dahingehend, dass ein Adapterkörper 17a vorgesehen ist, der anstelle eines Luer-Lock-Abschnittes einen in Form eines Luer-Slip-Abschnittes ausgestalteten Verbindungsabschnitt 16a aufweist. Insbesondere der Hohlzylinder 3 sowie der zur Verbindung mit dem Adapterkörper 17a vorgesehene Kragenabschnitt 25 sind unverändert. Demzufolge kann der Hohlzylinder 3 unabhängig von der Ausgestaltung des Verbindungsabschnittes 16, 16a hergestellt werden und umgekehrt. Hierdurch können fertigungs- und montageseitige Vorteile erreicht werden.

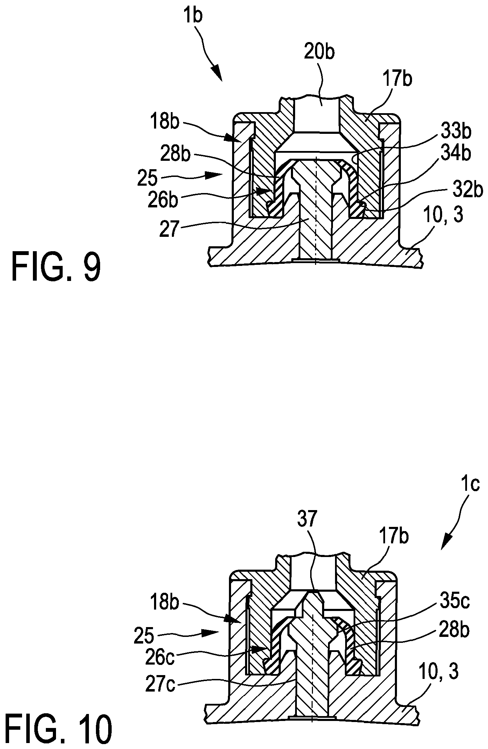

Demgegenüber unterscheidet sich die Injektionsvorrichtung 1b nach Fig. 9 im Wesentlichen dahingehend, dass eine abweichende Ausgestaltung des Formschlusses zwischen einem Dichtring 28b und einem Adapterkörper 17b vorgesehen ist. Genauer ist ein Passabschnitt 33b eines Lumens 20b mit einer hinterschnittenen Radialnut 34b versehen. Eine Radialnut 34b ist in proximale Richtung P einseitig offen, wobei sich der Nutgrund in proximaler Richtung P radial verjüngt. Ein Klemmabschnitt 32b des Dichtringes 28b ist entsprechend dem Nutverlauf der Radialnut 34b komplementär ausgestaltet und hintergreift diese somit in proximaler Richtung formschlüssig. Vor einem fertigungsseitigen Zusammenfügen des Adapterkörpers 17b mit dem Kragenabschnitt 25 des Hohlzylinders 3 kann der Dichtring 28b somit verliersicher an dem Adapterkörper 17b festgelegt werden. Dies erlaubt insbesondere eine vereinfachte Handhabung bei der Montage.

Die Injektionsvorrichtung 1c nach Fig. 10 unterscheidet sich von der Injektionsvorrichtung 1b nach Fig. 9 lediglich dahingehend, dass der dortige Ventilkörper 27c einen modifizierten Kopfabschnitt 35c aufweist. Der Kopfabschnitt 35c weist einen in distaler Richtung erstreckten und radial verjüngten Fortsatz 37 auf.

## Patentansprüche

1. Medizinische Injektionsvorrichtung (1, 1a, 1b, 1c) zur Einmalverwendung bei der Verabreichung einer Flüssigkeit (2) mit einem Hohlzylinder (3), der einen Hohlraum (4) zur Aufnahme der Flüssigkeit (2) sowie ein distales Ende (8) aufweist, einem im Bereich des distalen Endes (8) des Hohlzylinders (3) angeordneten Durchlass (9), der fluidleitend mit dem Hohlraum (4) verbunden und zum Ein- und/oder Ausströmen der Flüssigkeit (2) aus dem Hohlraum (4) vorgesehen ist, einem Verbindungsabschnitt (16, 16a) zur lösbaren fluiddichten Verbindung mit einem komplementären Gegenverbindungsabschnitt einer Injektionskomponente (12), der derart angeordnet ist, dass der Durchlass (9) fluidleitend mit einem Lumen (15) der Injektionskomponente (12) verbindbar ist, und einer Sperrventilanordnung (26, 26b, 26c), die einen Ventilkörper (27, 27c), welcher an seinem distalen Ende einen radial aufgeweiteten Kopfabschnitt (35, 35c) aufweist, und einen weichelastischen Dichtring (28, 28b) mit einer Öffnung (29) aufweist und derart angeordnet und/oder gestaltet ist, dass der Ventilkörper (27, 27c) mittels einer distal durch den Durchlass (9) gerichteten Strömung der Flüssigkeit (2) ausgehend von einer Freigabeposition, in welcher der Durchlass (9) freigegeben ist, wenigstens abschnittsweise in distale Richtung (D) durch die Öffnung (29) des Dichtringes (28, 28b) in eine Sperrposition beweglich ist, in welcher der radial aufgeweitete Kopfabschnitt (35, 35c) des Ventilkörpers (27, 27c) die Öffnung (29) derart hintergreift, dass eine Bewegung des Ventilkörpers (27, 27c) in die Freigabeposition gesperrt und der Durchlass (9) gegen eine proximal gerichtete Strömung abgedichtet ist, wobei ein Adapterkörper (17, 17a, 17b) mit einem proximalen Ende (18, 18b), das kraft- und/oder formschlüssig mit dem distalen Ende (8) des Hohlzylinders (3) verbunden ist, und einem distalen Ende (19, 19a), an dem der Verbindungsabschnitt (16, 16a) ausgebildet ist, und einem mit dem Durchlass (9) fluidleitend verbundenen Lumen (20, 20b) vorgesehen ist, wobei die Sperrventilanordnung (26, 26b, 26c) an dem proximalen Ende (18, 18b) des Adapterkörpers (17, 17a, 17b) und an dem distalen Ende (8) des Hohlzylinders (3) angeordnet ist, **dadurch gekennzeichnet, dass** der Hohlzylinder (3) einen einstückig angeformten Zylinderboden (10) aufweist, wobei der Ventilkörper (27, 27c) an einer durch den Zylinderboden (10) erstreckten Führungsbahn (30) axial beweglich geführt ist.

2. Medizinische Injektionsvorrichtung (1, 1a, 1b, 1c) nach Anspruch 1, **dadurch gekennzeichnet, dass** der Dichtring (28, 28b) einen Klemmabschnitt (32, 32b) aufweist, der zwischen dem Hohlzylinder (3), insbesondere dem Zylinderboden (10), und dem Adapterkörper (17, 17a, 17b) in Axialrichtung und/oder in Radialrichtung formschlüssig festgelegt, insbesondere geklemmt ist.

3. Medizinische Injektionsvorrichtung (1, 1a, 1b, 1c) nach einem der vorhergehenden Ansprüche, **dadurch gekennzeichnet, dass** das Lumen (20, 20b) des Adapterkörpers (17, 17a, 17b) einen proximal angeordneten Passabschnitt (33, 33b) aufweist, in dem der Dichtring (28, 28b) eingepasst ist.

4. Medizinische Injektionsvorrichtung (1b, 1c) nach Anspruch 3, **dadurch gekennzeichnet, dass** der Passabschnitt (33b) eine einseitig in proximale Richtung (P) offene, hinterschnittene Radialnut (34b) aufweist, wobei der Dichtring (28b) die Radialnut (34b) hintergreift und somit in proximaler Richtung (P) formschlüssig an dem Adapterkörper (17b) festgelegt ist.

5. Medizinische Injektionsvorrichtung (1, 1a, 1b, 1c) nach einem der vorhergehenden Ansprüche, **dadurch gekennzeichnet, dass** das distale Ende (8) des Hohlzylinders (3) einen Rastabschnitt (21) aufweist, der derart mit einem komplementären Gegenrastabschnitt (22) des Adapterkörpers (17, 17a, 17b), vorzugsweise unlösbar, verrastet ist, dass der Adapterkörper (17, 17a, 17b) in Axialrichtung an dem Hohlzylinder (3) festgelegt ist.

6. Medizinische Injektionsvorrichtung (1, 1a, 1b, 1c) nach einem der vorhergehenden Ansprüche, **dadurch gekennzeichnet, dass** das distale Ende (8) des Hohlzylinders (3) einen Profilabschnitt (23), vorzugsweise in Form einer Vielverzahnung, aufweist, der derart mit einem komplementären Gegenprofilabschnitt (24) des Adapterkörpers (17, 17a, 17b) verzahnt ist, dass der Adapterkörper (17, 17a, 17b) in Umfangsrichtung an dem Hohlzylinder (3) festgelegt ist.

7. Medizinische Injektionsvorrichtung (1, 1a, 1b, 1c) nach Anspruch 5 oder 6, **dadurch gekennzeichnet, dass** der Rastabschnitt (21) und/oder der Profilabschnitt (23) an einem umlaufenden und distal zu dem Hohlraum (4), insbesondere dem Zylinderboden (10) erstreckten Kragenabschnitt (25) des Hohlzylinders (3) ausgebildet ist.

8. Medizinische Injektionsvorrichtung (1c) nach einem der vorhergehenden Ansprüche, **dadurch gekennzeichnet, dass** der Kopfabschnitt (35c) einen in distale Richtung (D) erstreckten und radial verjüngten Fortsatz (37) aufweist.

## Claims

1. Medical injection device (1, 1a, 1b, 1c) for single use in the administration of a liquid (2), with a hollow cylinder (3), which has a cavity (4) for receiving the liquid (2) and has a distal end (8), with a passage (9) which is arranged in the region of the distal end (8) of the hollow cylinder (3) and is fluidically connected to the cavity (4) and is provided for the inflow and/or outflow of the liquid (2) from the cavity (4), with a connection portion (16, 16a) for releasable fluid-tight connection to a complementary mating connection portion of an injection component (12) arranged in such a way that the passage (9) can be fluidically connected to a lumen (15) of the injection component (12), and with a shut-off valve arrangement (26, 26b, 26c) which has a valve body (27, 27c), the valve body (27, 27c) having a radially widened head portion (35, 35c) at its distal end, and a flexible sealing ring (28, 28b) with an opening (29) and which is arranged and/or configured in such a way that, by means of a flow of the liquid (2) directed distally through the passage (9), the valve body (27, 27c), proceeding from a release position in which the passage (9) is freed, is at least partially movable in the distal direction (D) through the opening (29) of the sealing ring (28, 28b) to a blocking position in which the radially widened head portion (35, 35c) of the valve body (27, 27c) engages behind the opening (29) in such a way that a movement of the valve body (27, 27c) to the release position is blocked and the passage (9) is sealed off against a proximally directed flow, wherein an adapter body (17, 17a, 17b) is provided having a proximal end (18, 18b), which is connected with force-fit and/or form-fit engagement to the distal end (8) of the hollow cylinder (3), and a distal end (19, 19a), on which the connection portion (16, 16a) is formed, and having a lumen (20, 20b) fluidically connected to the passage (9), wherein the shut-off valve arrangement (26, 26b, 26c) is arranged at the proximal end (18, 18b) of the adapter body (17, 17a, 17c) and at the distal end (8) of the hollow cylinder (3), **characterized in that** the hollow cylinder (3) has an integrally moulded cylinder base (10), wherein the valve body (27, 27c) is guided axially movably on a guide path (30) extending through the cylinder base (10) .

2. Medical injection device (1, 1a, 1b, 1c) according to Claim 1, **characterized in that** the sealing ring (28, 28b) has a clamping portion (32, 32b) which is fixed by form-fit engagement, in particular clamped, in the axial direction and/or in the radial direction between the hollow cylinder (3), in particular the cylinder base (10), and the adapter body (17, 17a, 17b).

3. Medical injection device (1, 1a, 1b, 1c) according to one of the preceding claims, **characterized in that** the lumen (20, 20b) of the adapter body (17, 17a, 17b) has a proximally arranged receiving portion (33, 33b) in which the sealing ring (28, 28b) is fitted.

4. Medical injection device (1b, 1c) according to Claim 3, **characterized in that** the receiving portion (33b) has an undercut radial groove (34b) open at one side in the proximal direction (P), wherein the sealing ring (28b) engages behind the radial groove (34b) and is thus fixed with form-fit engagement on the adapter body (17b) in the proximal direction (P).

5. Medical injection device (1, 1a, 1b, 1c) according to one of the preceding claims, **characterized in that** the distal end (8) of the hollow cylinder (3) has a latch portion (21) which latches with a complementary mating latch portion (22) of the adapter body (17, 17a, 17b), preferably non-releasably, in such a way that the adapter body (17, 17a, 17b) is fixed in the axial direction on the hollow cylinder (3).

6. Medical injection device (1, 1a, 1b, 1c) according to one of the preceding claims, **characterized in that** the distal end (8) of the hollow cylinder (3) has a profile portion (23), preferably in the form of a multi-tooth arrangement, which meshes with a complementary mating profile portion (24) of the adapter body (17, 17a, 17b) in such a way that the adapter body (17, 17a, 17b) is fixed in the circumferential direction on the hollow cylinder (3) .

7. Medical injection device (1, 1a, 1b, 1c) according to Claim 5 or 6, **characterized in that** the latch portion (21) and/or the profile portion (23) is formed on a circumferential collar portion (25) of the hollow cylinder (3), which collar portion (25) extends distally with respect to the cavity (4), in particular with respect to the cylinder base (10) .

8. Medical injection device (1c) according to one of the preceding claims , **characterized in that** the head portion (35c) has a radially tapered continuation (37) extending in the distal direction (D).

## Revendications

1. Dispositif médical d'injection (1, 1a, 1b, 1c) à usage unique pour l'administration d'un liquide (2) avec un cylindre creux (3) qui présente une cavité (4) pour recevoir le liquide (2) ainsi qu'une extrémité distale (8), un passage (9) agencé dans la zone de l'extrémité distale (8) du cylindre creux (3), qui est relié fluidiquement à la cavité (4) et qui est prévu pour l'entrée et/ou la sortie du liquide (2) de la cavité (4), une section de liaison (16, 16a) pour la liaison amovible étanche aux fluides avec une section de liaison opposée complémentaire d'un composant d'injection (12), qui est agencée de telle sorte que le passage (9) peut être relié fluidiquement à une lumière (15) du composant d'injection (12), et un agencement de soupape d'arrêt (26, 26b, 26c) qui présente un corps de soupape (27, 27c) qui présente à son extrémité distale une section de tête élargie radialement (35, 35c), et une bague d'étanchéité élastique souple (28, 28b) avec une ouverture (29), et est agencé et/ou conçu de telle sorte que le corps de soupape (27, 27c), au moyen d'un écoulement du liquide (2) dirigé de manière distale à travers le passage (9), en partant d'une position de dégagement, dans laquelle le passage (9) est dégagé, peut être déplacé au moins par sections dans la direction distale (D) à travers l'ouverture (29) de la bague d'étanchéité (28, 28b) dans une position de blocage dans laquelle la section de tête élargie radialement (35, 35c) du corps de soupape (27, 27c) s'engage derrière l'ouverture (29) de telle sorte qu'un mouvement du corps de soupape (27, 27c) dans la position de dégagement est bloqué et que le passage (9) est étanchéifié contre un écoulement dirigé de manière proximale, il étant prévu un corps d'adaptateur (17, 17a, 17b) avec une extrémité proximale (18, 18b), qui est reliée par adhérence et/ou par complémentarité de forme à l'extrémité distale (8) du cylindre creux (3), et une extrémité distale (19, 19a), sur laquelle est réalisée la section de liaison (16, 16a), et une lumière (20, 20b) reliée fluidiquement au passage (9), l'agencement de soupape d'arrêt (26, 26b, 26c) étant agencé à l'extrémité proximale (18, 18b) du corps d'adaptateur (17, 17a, 17b) et à l'extrémité distale (8) du cylindre creux (3), **caractérisé en ce que** le cylindre creux (3) présente un fond de cylindre (10) formé d'une seule pièce, le corps de soupape (27, 27c) étant guidé de manière mobile axialement sur une voie de guidage (30) s'étendant à travers le fond de cylindre (10).

2. Dispositif médical d'injection (1, 1a, 1b, 1c) selon la revendication 1, **caractérisé en ce que** la bague d'étanchéité (28, 28b) présente une section de serrage (32, 32b), qui est fixée, notamment serrée, entre le cylindre creux (3), notamment le fond de cylindre (10), et le corps d'adaptateur (17, 17a, 17b) dans la direction axiale et/ou dans la direction radiale, par complémentarité de forme.

3. Dispositif médical d'injection (1, 1a, 1b, 1c) selon l'une quelconque des revendications précédentes, **caractérisé en ce que** la lumière (20, 20b) du corps d'adaptateur (17, 17a, 17b) présente une section d'encastrement (33, 33b) agencée de manière proximale, dans laquelle la bague d'étanchéité (28, 28b) est encastrée.

4. Dispositif médical d'injection (1b, 1c) selon la revendication 3, **caractérisé en ce que** la section d'encastrement (33b) présente une rainure radiale (34b) en contre-dépouille, ouverte d'un côté dans la direction proximale (P), la bague d'étanchéité (28b) s'engageant derrière la rainure radiale (34b) et étant ainsi fixée par complémentarité de forme dans la direction proximale (P) sur le corps d'adaptateur (17b).

5. Dispositif médical d'injection (1, 1a, 1b, 1c) selon l'une quelconque des revendications précédentes, **caractérisé en ce que** l'extrémité distale (8) du cylindre creux (3) présente une section d'encliquetage (21) qui est encliquetée, de préférence de manière inamovible, avec une section d'encliquetage opposée complémentaire (22) du corps d'adaptateur (17, 17a, 17b) de telle sorte que le corps d'adaptateur (17, 17a, 17b) est fixé dans la direction axiale sur le cylindre creux (3).

6. Dispositif médical d'injection (1, 1a, 1b, 1c) selon l'une quelconque des revendications précédentes, **caractérisé en ce que** l'extrémité distale (8) du cylindre creux (3) présente une section profilée (23), de préférence sous la forme d'une denture multiple, qui est imbriquée avec une section profilée opposée complémentaire (24) du corps d'adaptateur (17, 17a, 17b) de telle sorte que le corps d'adaptateur (17, 17a, 17b) est fixé dans la direction circonférentielle sur le cylindre creux (3).

7. Dispositif médical d'injection (1, 1a, 1b, 1c) selon la revendication 5 ou 6, **caractérisé en ce que** la section d'encliquetage (21) et/ou la section profilée (23) est réalisée sur une section de collerette (25) du cylindre creux (3), périphérique et s'étendant de manière distale par rapport à la cavité (4), notamment au fond de cylindre (10) .

8. Dispositif médical d'injection (1c) selon l'une quelconque des revendications précédentes, **caractérisé en ce que** la section de tête (35c) présente un prolongement (37) s'étendant dans la direction distale (D) et se rétrécissant radialement.
